# FASCICULE DE BREVET EUROPEEN

(11) **EP 1 867 350 B1**
(45) Date de publication et mention de la délivrance du brevet: **24.09.2008**
(21) Numéro de dépôt: 07290723.1
(22) Date de dépôt: 11.06.2007
(51) Int. Cl.: A61M 1/10, A61M 1/12

(54) **Dispositif de connexion rapide entre une prothèse cardiaque totalement implantable et des oreillettes naturelles**
Schnellverbindungsvorrichtung zwischen einer voll implantierbaren Herzprothese und natürlichen Herzvorhöfen
Quick-connection device between a completely implantable artificial heart and natural atria

(30) Priorité: 15.06.2006 FR 0605331
(43) Date de publication de la demande: 19.12.2007
(73) Titulaire: Groupement Coeur Artificiel Total Carpentier Matra Carmat, 78140 Velizy Villacoublay (FR)
(72) Inventeur: Parquet, Jean-Marc, 95330 Domont (FR); Carpentier, Alain, 75116 Paris (FR)
(74) Mandataire: Bonnetat, Christian

(56) Documents cités:
- EP-A1- 0 324 669
- US-A- 5 089 014

## Description

La présente invention concerne un dispositif de connexion rapide entre une prothèse cardiaque totalement implantable et des oreillettes naturelles.

Par le document US-5 135 539, on connaît déjà une prothèse cardiaque de ce type, implantable dans la cavité péricardique d'un patient et apte à remplacer les ventricules gauche et droit naturels dudit patient, après ablation de ceux-ci. Cette prothèse cardiaque comporte un corps rigide dans lequel sont agencés des ventricules gauche et droit artificiels, lesdits ventricules artificiels étant pourvus de moyens de raccord rapide aux oreillettes naturelles gauche et droite dudit patient comportant :
- une première lunette, faisant partie intégrante dudit corps rigide et comportant des premier et deuxième orifices, respectivement en communication avec le ventricule artificiel gauche et avec le ventricule artificiel droit, par l'intermédiaire de valves ; et
- une seconde lunette comportant des troisième et quatrième orifices aptes à être respectivement reliés à ladite oreillette naturelle gauche et à ladite oreillette naturelle droite.

Dans cette réalisation, lesdites première et seconde lunettes sont aptes à être raccordées l'une à l'autre, de façon amovible, pour prendre une position de fonctionnement dans laquelle se trouvent respectivement en regard, d'une part, lesdits premier et troisième orifices et, d'autre part, lesdits deuxième et quatrième orifices.

Un tel système de lunettes facilite grandement le raccordement de ladite prothèse sur ladite seconde lunette, préalablement raccordée auxdites oreillettes naturelles. Cependant, du fait de la disposition de la prothèse cardiaque dans la cavité péricardique au moment de l'implantation, ce raccordement s'effectue en aveugle et rien ne garantit que la position de raccord obtenue est précise et correspond exactement à ladite position de fonctionnement.

La présente invention a pour objet de perfectionner la prothèse cardiaque décrite dans le document US-5 135 539 pour remédier à cet inconvénient.

A cette fin, selon l'invention, la prothèse cardiaque implantable dans la cavité péricardique d'un patient, ladite prothèse étant apte à remplacer les ventricules gauche et droit naturels dudit patient après ablation de ceux-ci et comportant un corps rigide dans lequel sont agencés des ventricules artificiels gauche et droit, lesdits ventricules artificiels étant pourvus de moyens de raccord aux oreillettes naturelles gauche et droite dudit patient comportant :
- une première lunette, faisant partie intégrante dudit corps rigide et comportant des premier et deuxième orifices, respectivement en communication avec le ventricule artificiel gauche et avec le ventricule artificiel droit ; et
- une seconde lunette comportant des troisième et quatrième orifices aptes à être respectivement reliés à ladite oreillette naturelle gauche et à ladite oreillette naturelle droite,
lesdites première et seconde lunettes étant aptes à être raccordées l'une à l'autre, de façon amovible, pour prendre une position de fonctionnement dans laquelle se trouvent respectivement en regard, d'une part, lesdits premier et troisième orifices et, d'autre part, lesdits deuxième et quatrième orifices,
est remarquable en ce qu'elle comporte :
- des moyens d'accrochage d'une extrémité de l'une des lunettes sur l'autre lunette, lesdits moyens d'accrochage étant aptes à permettre le rabattement desdites lunettes l'une sur l'autre ;
- des moyens de guidage desdites lunettes l'une par rapport à l'autre aussi bien avant et pendant l'accrochage que pendant le rabattement ; et
- des moyens d'encliquetage desdites lunettes l'une sur l'autre dans ladite position de fonctionnement correspondant à la fin dudit rabattement.

Ainsi, selon l'invention, par la coopération desdits moyens d'accrochage, desdits moyens de guidage et desdits moyens d'encliquetage, non seulement le raccordement en aveugle de ladite prothèse sur ladite seconde lunette est simplifié, mais encore l'encliquetage ne peut se produire que si le positionnement relatif desdites lunettes correspond avec précision à ladite position de fonctionnement.

De préférence, lesdits moyens d'encliquetage se trouvent, par rapport auxdites lunettes, à l'opposé desdits moyens d'accrochage. Lesdits moyens d'encliquetage peuvent comporter des crochets élastiques portés par l'une desdites lunettes et des moyens d'ancrage desdits crochets élastiques portés par l'autre desdites lunettes, lesdits moyens d'ancrage étant amovibles par rapport à la lunette qui les porte. Ainsi, par retrait desdits moyens d'ancrage est-il possible, après encliquetage, de désolidariser ladite seconde lunette de la prothèse si cela s'avère nécessaire.

Avantageusement, lesdits moyens d'ancrage sont constitués par une goupille transversale apte à coulisser dans ladite lunette qui la porte.

Pour assurer un accrochage stable de ladite seconde lunette sur la première, il est avantageux que lesdits moyens d'accrochage comportent un axe transversal porté par l'une desdites lunettes et une rainure transversale qui est pratiquée dans l'autre desdites lunettes et dans laquelle peut se loger ledit axe transversal.

Lesdits moyens de guidage peuvent comporter au moins une face longitudinale sur ladite première lunette et au moins une face longitudinale sur ladite seconde lunette, lesdites faces longitudinales desdites première et seconde lunettes devant venir au contact l'une de l'autre avant l'accrochage et glissant l'une sur l'autre pendant ledit accrochage et ledit rabattement.

Dans un mode de réalisation préféré de la présente invention, lesdites première et seconde lunettes présentent chacune la forme générale d'un parallélépipède rectangle, ladite seconde lunette étant apte à servir de couvercle latéralement ajusté à ladite première lunette. Ainsi, dans ce cas, les deux faces longitudinales de ladite seconde lunette peuvent coopérer avec les deux faces longitudinales de ladite première lunette pour servir de moyens de guidage.

A l'une de ses extrémités, ladite goupille transversale constituant les moyens d'ancrage peut être pourvue d'une tête de préhension comportant des moyens, par exemple des méplats, permettant de saisir fermement ladite goupille, sans possibilité de rotation, avec une pince de chirurgie. De plus, on peut prévoir des moyens pour attacher ladite goupille à la lunette qui la porte pendant les manipulations de raccordement desdites lunettes.

Les figures du dessin annexé feront bien comprendre comment l'invention peut être réalisée. Sur ces figures, des références identiques désignent des éléments semblables.

La figure 1 est une vue schématique en perspective éclatée d'une prothèse cardiaque conforme à la présente invention.

Les figures 2A, 2B, 2C et 2D illustrent schématiquement en coupe longitudinale trois étapes de l'assemblage desdits lunettes.

La figure 3 est une vue en perspective de la prothèse cardiaque de l'invention dans une position correspondant à la figure 2C.

La figure 4 est une vue en perspective partielle correspondant à la flèche IV de la figure 2D.

La figure 5 est une vue en perspective du dessus correspondant à la figure 2D, la goupille d'encliquetage n'étant pas complètement engagée à des fins de clarté de dessin.

La figure 6 est une vue en perspective partielle du dessous avec arrachements, illustrant l'encliquetage amovible desdites lunettes.

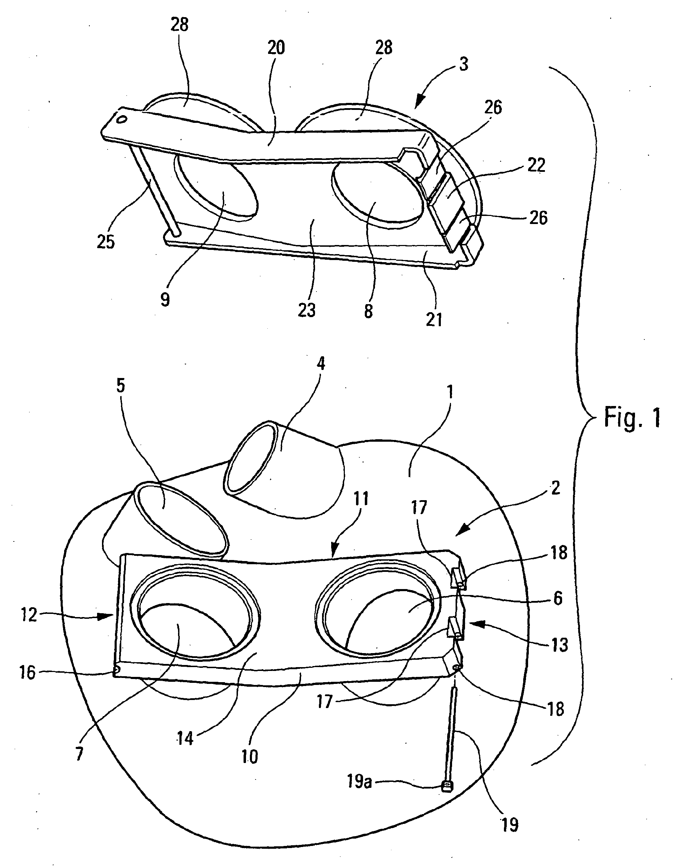

La prothèse cardiaque conforme à la présente invention, représentée sur la figure 1 de façon éclatée, est apte à remplacer les ventricules gauche et droit naturels d'un patient, après leur ablation. Elle comporte un corps rigide 1 dans lequel sont agencés des ventricules gauche et droit artificiels (non visibles sur la figure 1), lesdits ventricules étant pourvus de moyens de raccord 2, 3 aux oreillettes gauche et droite naturelles dudit patient (non visibles), ainsi que de moyens de raccord 4 et 5, respectivement à l'aorte et à l'artère pulmonaire.

Les moyens de raccord auxdites oreillettes gauche et droite naturelles comportent :
- une lunette 2 faisant partie intégrante du corps rigide 1 et pourvue d'un orifice 6 en communication avec le ventricule artificiel droit et d'un orifice 7 en communication avec le ventricule artificiel gauche, des valves (non représentées sur la figure 1) étant disposées dans lesdits orifices 6, 7 ; et
- une lunette 3 sur laquelle peut être raccordée la lunette 2 de façon amovible, ladite lunette 3 comportant des orifices 8 et 9 destinés à être disposés respectivement en regard desdits orifices 6 et 7, lorsque lesdites lunettes 2 et 3 occupent la position relative précise correspondant au fonctionnement de la prothèse.

La lunette 2 présente la forme générale d'un parallélépipède rectangle pourvu de deux faces latérales longitudinales 10 et 11 parallèles, d'une face arrière 12, d'une face avant 13 et d'une face supérieure 14.

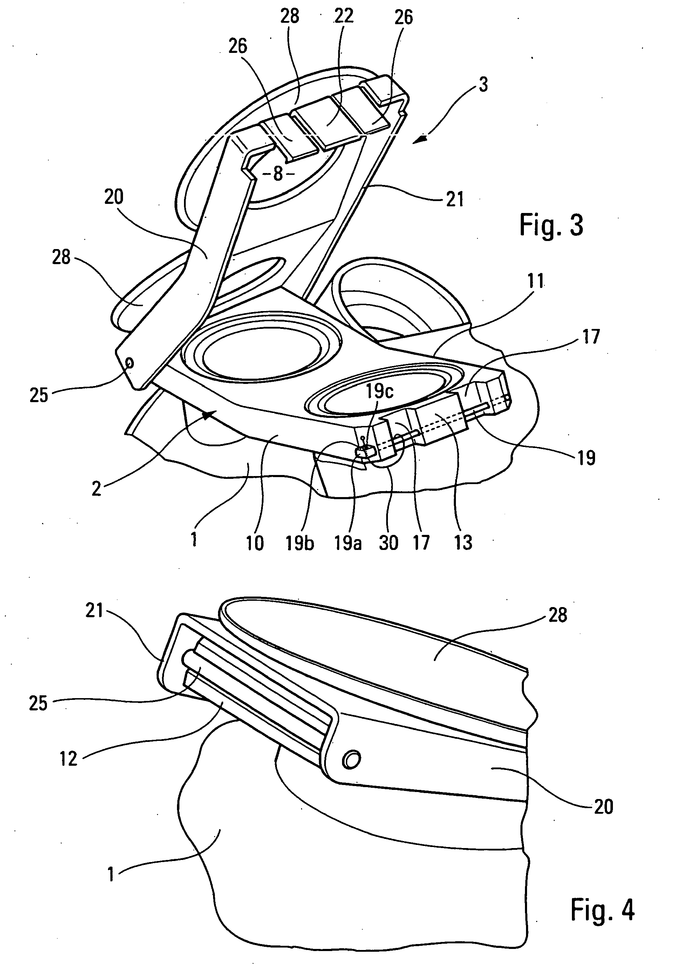

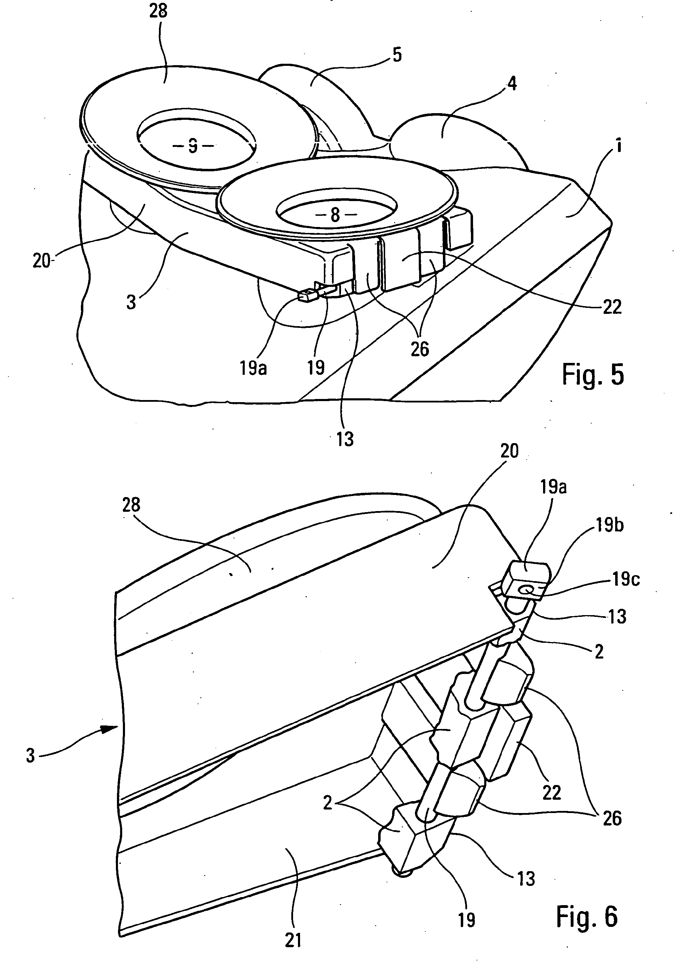

La face arrière 12 comporte une rainure 16, transversale par rapport à ladite lunette 2, tandis que la face avant 1 3 comporte des échancrures 17. Par ailleurs, au niveau de ladite face avant 13, la lunette 2 est percée par un conduit transversal 18, traversant lesdites échancrures 17 et apte à recevoir une goupille amovible 19. Ainsi, lorsque ladite goupille est mise en place dans le conduit 18, elle est disposée transversalement à ladite lunette 2 et elle est apparente dans la traversée desdites échancrures 17 (voir les figures 3 et 6).

La lunette 3 présente la forme générale de la lunette 2, à laquelle elle peut servir de couvercle. Ladite lunette 3 est pourvue de deux faces latérales longitudinales 20 et 21 parallèles, d'une face avant 22 et d'une face supérieure 23. La face arrière de la lunette 3 est ouverte et traversée par un axe transversal 25, tandis que la face avant 22 comporte un ou des crochets élastiques 26, respectivement en regard de la ou des échancrures 17. Le ou lesdits crochets élastiques peuvent être rapportés à ladite lunette 3 ou bien, comme représenté sur les figures, être des parties intégrantes de cette dernière, lorsqu'elle est réalisée en une matière élastique, par exemple synthétique.

Sur la figure 1, on a de plus représenté, sur la lunette 3, des collerettes de suture individuelles 28 équipant les orifices 8 et 9.

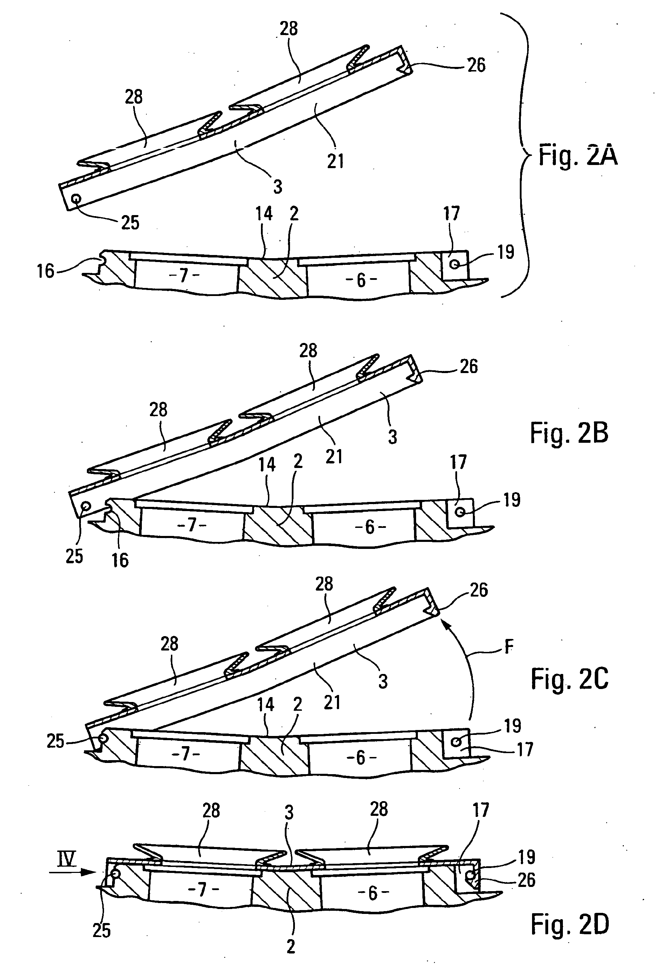

Comme l'illustrent schématiquement les figures 2A, 2B, 2C et 2D, la mise en place du corps 1 sur la lunette 3 est particulièrement aisée et précise, après mise en place de la goupille 19 dans le conduit 18 :
- tout d'abord, la lunette 2 est approchée de la lunette 3, de façon que la rainure transversale 16 soit du côté, et proche, de l'axe transversal 25 (voir la figure 2A) ;
- le rapprochement des lunettes 2 et 3 est poursuivi jusqu'à ce que les extrémités arrière en regard des faces longitudinales 10, 11 et 20, 21 coopèrent les unes avec les autres pour servir de guidage (voir la figure 2B) ;
- ensuite, l'axe transversal 25 est accroché dans -la rainure transversale 16, cet accrochage n'étant possible que si les lunettes 2 et 3 sont centrées l'une par rapport à l'autre; ce qui est obtenu par la coopération desdites extrémités arrière des faces longitudinales 20 et 21 de la lunette 3 et des extrémités arrière des faces longitudinales 10 et 11 de la lunette 2. Les lunettes 2 et 3 se trouvent alors dans la position relative illustrée par les figures 2C et 3 ;
- finalement, comme indiqué par la flèche F de la figure 2C, la lunette 2 est rabattue sur la lunette 3 de façon que le ou les crochets 26 pénètrent dans la ou les échancrures 17 et viennent se verrouiller élastiquement sur la goupille 19. Pendant ce rabattement, les lunettes 2 et 3 sont guidées l'une par rapport à l'autre, par la coopération des faces longitudinales 10, 11, 20 et 21. On se trouve alors dans la position de fonctionnement schématisée par la figure 2D et illustrée par les figures 4, 5 et 6. On remarquera que la face avant 22, comprise entre les crochets élastiques 26, est rigide et interdit l'encliquetage des lunettes 2 et 3 si l'axe 25 n'est pas correctement engagé dans la rainure 16. Cela garantit un bon positionnement axial des lunettes et évite des contraintes élevées sur les crochets 26.

A partir de cette position de fonctionnement, il est aisé, en cas de nécessité, de désassembler les lunettes 2 et 3 en sortant la goupille 19 du conduit 18. En effet, on supprime alors l'ancrage du ou des crochets 26 sur la lunette 2, de sorte que le corps 1 peut être basculé autour de l'axe 25, puis séparé de la lunette 3.

Afin de faciliter les manipulations de la goupille 19, son extrémité de préhension 19a est étudiée de manière à empêcher toute rotation, lorsqu'elle est saisie à l'aide d'une pince de chirurgie. Elle comporte à cet effet au moins deux méplats 19b diamétralement opposés. De plus, pour éviter que ladite goupille ne sorte de son logement 18 pendant les manipulations de la lunette 2, son extrémité de préhension est percée de part en part par un orifice 1 9c traversé par un lien 30, apte à attacher ladite goupille 1 9 à la lunette 2 (voir figure 3).

## Revendications

1. Prothèse cardiaque implantable dans la cavité péricardique d'un patient, ladite prothèse étant apte à remplacer les ventricules gauche et droit naturels dudit patient après ablation de ceux-ci et comportant un corps rigide (1) dans lequel sont agencés des ventricules artificiels gauche et droit, lesdits ventricules artificiels étant pourvus de moyens de raccord aux oreillettes gauche et droite naturelles dudit patient comportant :
- une première lunette (2), faisant partie intégrante dudit corps rigide (1) et comportant des premier et deuxième orifices (6, 7), respectivement en communication avec le ventricule artificiel gauche et avec le ventricule artificiel droit ; et
- une seconde lunette (3) comportant des troisième et quatrième orifices (8, 9) aptes à être respectivement reliés à ladite oreillette naturelle gauche et à ladite oreillette naturelle droite,
lesdites première et seconde lunettes (2, 3) étant aptes à être raccordées l'une à l'autre, de façon amovible, pour prendre une position de fonctionnement dans laquelle se trouvent respectivement en regard, d'une part, lesdits premier et troisième orifices (6, 8) et, d'autre part, lesdits deuxième et quatrième orifices (7, 9),
**caractérisée en ce qu'**elle comporte :
- des moyens d'accrochage (16, 25) d'une extrémité de l'une des lunettes sur l'autre lunette, lesdits moyens d'accrochage étant aptes à permettre le rabattement desdites lunettes l'une sur l'autre ;
- des moyens de guidage (10, 11, 20, 21, 22) desdites lunettes l'une par rapport à l'autre aussi bien avant et pendant l'accrochage que pendant le rabattement ; et
- des moyens d'encliquetage (19, 26) desdites lunettes l'une sur J'autre dans ladite position de fonctionnement correspondant à la fin dudit rabattement.

2. Prothèse cardiaque selon la revendication 1,
**caractérisée en ce que** lesdits moyens d'encliquetage (19, 26) se trouvent, par rapport auxdites lunettes, à l'opposé desdits moyens d'accrochage (16, 25).

3. Prothèse cardiaque selon l'une des revendications 1 ou 2,
**caractérisée en ce que** lesdits moyens d'encliquetage comportent un ou des crochets élastiques (26) portés par l'une desdites lunettes et des moyens d'ancrage (19) desdits crochets élastiques portés par l'autre desdites lunettes, lesdits moyens d'ancrage (19) étant amovibles par rapport à la lunette qui les porte.

4. Prothèse cardiaque selon la revendication 3,
**caractérisée en ce que** lesdits moyens d'ancrage amovibles (19) sont constitués par une goupille apte à coulisser transversalement dans ladite lunette qui la porte.

5. Prothèse cardiaque selon l'une des revendications 1 à 4,
**caractérisée en ce que** lesdits moyens d'accrochage comportent un axe transversal (25) porté par l'une desdites lunettes (3) et une rainure transversale (16) qui est pratiquée dans l'autre desdites lunettes (2) et dans laquelle peut se loger ledit axe transversal (25).

6. Prothèse cardiaque selon l'une des revendications 1 à 5,
**caractérisée en ce que** lesdits moyens de guidage comportent au moins une face longitudinale (10, 11) sur ladite première lunette (2) et au moins une face longitudinale (20, 21) sur ladite seconde lunette (3), lesdites faces longitudinales desdites première et seconde lunettes devant venir au contact l'une de l'autre avant l'accrochage et glissant l'une sur l'autre pendant ledit accrochage et ledit rabattement.

7. Prothèse cardiaque selon l'une des revendications 1 à 6,
**caractérisée en ce que** lesdites première et seconde lunettes (2, 3) présentent chacune la forme générale d'un parallélépipède rectangle, ladite seconde lunette (3) étant apte à servir de couvercle latéralement ajusté à ladite première lunette (2).

8. Prothèse cardiaque selon l'une des revendications 4 à 7,
**caractérisée en ce que** l'extrémité de préhension (19a) de la goupille (19) comporte des moyens (19b) permettant de la saisir fermement avec une pince de chirurgie.

9. Prothèse cardiaque selon l'une des revendications 4 à 8,
**caractérisée en ce que** des moyens (19c, 30) sont prévus pour attacher ladite goupille (19) à la lunette qui la porte pendant les manipulations de raccordement desdites lunettes.

## Claims

1. Heart prosthesis implantable in the pericardial cavity of a patient, said prosthesis being able to replace the natural left and right ventricles of said patient after their removal, and comprising a rigid body (1) in which artificial left and right ventricles are arranged, said artificial ventricles being provided with means for connection to the natural left and right auricles of said patient, comprising:
- a first bezel (2) forming an integral part of said rigid body (1) and comprising first and second orifices (6, 7), which communicate respectively with the artificial left ventricle and with the artificial right ventricle; and
- a second bezel (3) comprising third and fourth orifices (8, 9) that can be connected respectively to said natural left auricle and to said natural right auricle,
said first and second bezels (2, 3) being able to be connected to each other removably, in order to assume an operative position in which said first and third orifices (6, 8) are located opposite each other and said second and fourth orifices (7, 9) are located opposite each other,
**characterized in that** it comprises:
- fastening means (16, 25) for fastening one end of one of the bezels to the other bezel, said fastening means being able to permit folding of said bezels relative to each other;
- guide means (10, 11, 20, 21, 22) for guiding said bezels relative to each other before and during fastening and also during folding; and
- snap-fit means (19, 26) for engaging said bezels on each other in said operative position corresponding to the end of said folding.

2. Heart prosthesis according to Claim 1, **characterized in that** said snap-fit means (19, 26) are situated, relative to said bezels, remote from said fastening means (16, 25).

3. Heart prosthesis according to one of Claims 1 and 2, **characterized in that** said snap-fit means comprise one or more elastic hooks (26) carried by one of said bezels, and anchoring means (19), for anchoring said elastic hooks, carried by the other of said bezels, said anchoring means (19) being removable from the bezel that carries them.

4. Heart prosthesis according to Claim 3, **characterized in that** said removable anchoring means (19) are formed by a pin that can slide transversely in said bezel that carries it.

5. Heart prosthesis according to one of Claims 1 to 4, **characterized in that** said fastening means comprise a transverse shaft (25) carried by one of said bezels (3), and a transverse groove (16) which is formed in the other of said bezels (2) and in which said transverse shaft (25) can lodge itself.

6. Heart prosthesis according to one of Claims 1 to 5, **characterized in that** said guide means comprise at least one longitudinal face (10, 11) on said first bezel (2) and at least one longitudinal face (20, 21) on said second bezel (3), said longitudinal faces of said first and second bezels coming into contact with one another before fastening, and sliding on one another during said fastening and said folding.

7. Heart prosthesis according to one of Claims 1 to 6, **characterized in that** said first and second bezels (2, 3) each have the general shape of a right-angled parallelepiped, said second bezel (3) being able to serve as a cover fitted laterally on said first bezel (2).

8. Heart prosthesis according to one of Claims 4 to 7, **characterized in that** the grip end (19a) of the pin (19) comprises means (19b) allowing it to be taken hold of securely by surgical forceps.

9. Heart prosthesis according to one of Claims 4 to 8, **characterized in that** means (19c, 30) are provided for attaching said pin (19) to the bezel carrying it during the maneuvers for connection of said bezels.

## Patentansprüche

1. Herzprothese, welche in die Perikardhöhle eines Patienten implantierbar ist, wobei die Prothese geeignet ist, die natürliche linke und rechte Herzkammer des Patienten nach Ablation dieser zu ersetzen, und wobei sie einen starren Körper (1) umfasst, in dem eine künstliche linke und rechte Herzkammer angeordnet sind, wobei die künstlichen Herzkammern mit Mitteln zum Anschluss an das natürliche linke und rechte Herzohr des Patienten versehen sind, umfassend:
- eine erste Brille (2), die integraler Bestandteil des starren Körpers (1) ist und eine erste und eine zweite Öffnung (6, 7) umfasst, die mit der künstlichen linken Herzkammer bzw. mit der künstlichen rechten Herzkammer in Verbindung stehen; und
- eine zweite Brille (3), die eine dritte und eine vierte Öffnung (8, 9) umfasst, die geeignet sind, mit dem natürlichen linken Herzohr bzw. dem natürlichen rechten Herzohr verbunden zu werden,
wobei die erste und die zweite Brille (2, 3) geeignet sind, auf lösbare Weise miteinander verbunden zu werden, um eine Betriebsposition einzunehmen, in der einerseits die erste und die dritte Öffnung (6, 8) bzw. andererseits die zweite und die vierte Öffnung (7, 9) einander gegenüberliegen,
**dadurch gekennzeichnet, dass** sie umfasst:
- Mittel zum Ankoppeln (16, 25) eines Endes einer der Brillen an die andere Brille, wobei die Ankopplungsmittel geeignet sind, das Aufeinanderklappen der Brillen zu ermöglichen,
- Mittel zum Führen (10, 11, 20, 21, 22) der Brillen in Bezug zueinander sowohl vor und während des Ankoppelns als auch während des Aufeinanderklappens; und
- Mittel zum gegenseitigen Einrasten (19, 26) der Brillen in der Betriebsposition, die dem Ende des Aufeinanderklappens entspricht.

2. Herzprothese nach Anspruch 1,
**dadurch gekennzeichnet, dass** sich die Einrastmittel (19, 26) in Bezug auf die Brillen gegenüber den Ankopplungsmitteln (16, 25) befinden.

3. Herzprothese nach einem der Ansprüche 1 oder 2,
**dadurch gekennzeichnet, dass** die Einrastmittel umfassen: einen oder mehrere elastische Haken (26), die von einer der Brillen getragen werden, und Mittel zur Verankerung (19) der elastischen Haken, die von der anderen der Brillen getragen werden, wobei die Verankerungsmittel (19) in Bezug auf die Brille, die sie trägt, lösbar sind.

4. Herzprothese nach Anspruch 3,
**dadurch gekennzeichnet, dass** die lösbaren Verankerungsmittel (19) durch einen Stift gebildet werden, der geeignet ist, in Querrichtung in der Brille zu gleiten, die ihn trägt.

5. Herzprothese nach einem der Ansprüche 1 bis 4,
**dadurch gekennzeichnet, dass** die Ankopplungsmittel umfassen: eine Querachse (25), die von einer der Brillen (3) getragen wird, und eine Querrille (16), die in der anderen der Brillen (2) gebildet ist und in die sich die Querachse (25) einfügen kann.

6. Herzprothese nach einem der Ansprüche 1 bis 5,
**dadurch gekennzeichnet, dass** die Führungsmittel mindestens eine Längsseite (10, 11) an der ersten Brille (2) und mindestens eine Längsseite (20, 21) an der zweiten Brille (3) umfassen, wobei die Längsseiten der ersten und zweiten Brille vor dem Ankoppeln miteinander in Kontakt kommen müssen und während des Ankoppelns und Aufeinanderklappens aneinander gleiten.

7. Herzprothese nach einem der Ansprüche 1 bis 6,
**dadurch gekennzeichnet, dass** die erste und zweite Brille (2, 3) jeweils die allgemeine Form eines rechtwinkligen Parallelepipeds aufweisen, wobei die zweite Brille (3) geeignet ist, als Deckel zu dienen, der seitlich auf die erste Brille (2) ausgerichtet ist.

8. Herzprothese nach einem der Ansprüche 4 bis 7,
**dadurch gekennzeichnet, dass** das Griffende (19a) des Stifts (19) Mittel (19b) umfasst, die es ermöglichen, ihn mit einer chirurgischen Zange fest zu ergreifen.

9. Herzprothese nach einem der Ansprüche 4 bis 8,
**dadurch gekennzeichnet, dass** Mittel (19c, 30) vorgesehen sind, um während der Handgriffe zur Verbindung der Brillen den Stift (19) an der Brille zu befestigen, die ihn trägt.
